# EUROPEAN PATENT APPLICATION

(11) **EP 4 212 878 A1**
(43) Date of publication of application: **19.07.2023**
(21) Application number: 23150670.0
(22) Date of filing: 09.01.2023
(51) Int. Cl.: G01N 33/68

(54) **IMPROVED ANTIBODY MANUFACTURE**

(30) Priority: 13.01.2022 WO PCT/US2022/012215
(71) Applicant: Bayer Healthcare LLC, Whippany, NJ 07981 (US)
(72) Inventor: Tjandra, Hendri, Union City, CA, 94587 (US); Liu, Yi, Cupertino, CA, 95014 (US); Thai, Vu, San Rafael, CA, 94903 (US)
(74) Representative: BIP Patents

(57) **Abstract**

Described herein is a method for assessing disulfide bond reduction potential of a protein of interest comprising the following steps:
- Providing a cell culture fluid sample comprising mammalian cells expressing a protein of interest at a concentration within the range of between 0.2 g/l to 10 g/l
- Filtering said cell culture fluid sample over at least one filter
- Displacing O₂ in the filtered cell culture fluid sample
- Collecting at least one sample of the O₂-displaced filtered cell culture fluid sample
- Separating the proteins in said at least one O₂-displaced, filtered cell culture fluid sample according to their size under non-denaturing conditions
- Determining the disulfide bond reduction potential of the protein of interest.

## Description

Proteins of interest such as biological therapeutics e.g. recombinant proteins and monoclonal antibodies (mAbs) are usually manufactured by expression in cell cultures i.e. in host cells. A variety of cell types, including both prokaryotes and eukaryotes, can be used to express the protein of interest. The biopharmaceutical industry relies heavily on mammalian cells such as Chinese Hamster Ovary (CHO) cells due to their ability to produce properly folded and post-translationally modified proteins. Manufacturing a protein of interest such as a therapeutic protein typically begins by culturing cells in a bioreactor. Various nutrients and process parameters are monitored to ensure optimal cell growth and protein production. The protein of interest is typically secreted into the cell culture fluid where it accumulates. Thus the first step in harvesting the protein of interest is to remove the cells. Therefore, to recover the accumulated protein of interest, clarification techniques such centrifugation or filtration of the cell culture fluid are used. The protein of interest is subsequently further purified using appropriate affinity resin(s) or relevant ionic exchange chromatography resin(s). However, sometimes host cells may lyse before or during the clarification process, thus releasing cellular components that may alter or degrade the protein of interest. For example, enzymes and other components in the harvest cell culture may reduce disulfide bonds especially during storage of centrifuged or filtered cell culture fluid. Because intact disulfide bonds are critical for protein structure and biological functions, methods to predict and prevent disulfide reduction are of great interest.

### SUMMARY OF THE INVENTION

In a first aspect what is described herein relates to a method for assessing disulfide bond reduction potential of a protein of interest comprising the following steps:
- Providing a cell culture fluid sample comprising mammalian cells expressing a protein of interest at a concentration within the range of between 0.2 g/l to 10 g/l
- Filtering said cell culture fluid sample over at least one filter
- Displacing O₂ in the filtered cell culture fluid sample
- Collecting at least one sample of the O₂-displaced filtered cell culture fluid sample
- Separating the proteins in said at least one O₂-displaced, filtered cell culture fluid sample according to their size under non-denaturing conditions
- Determining the disulfide bond reduction potential of the protein of interest

In a second aspect what is described herein relates to the use of the method described herein for assessing disulfide bond reduction potential of a protein of interest.

### DESCRIPTION OF THE DRAWINGS

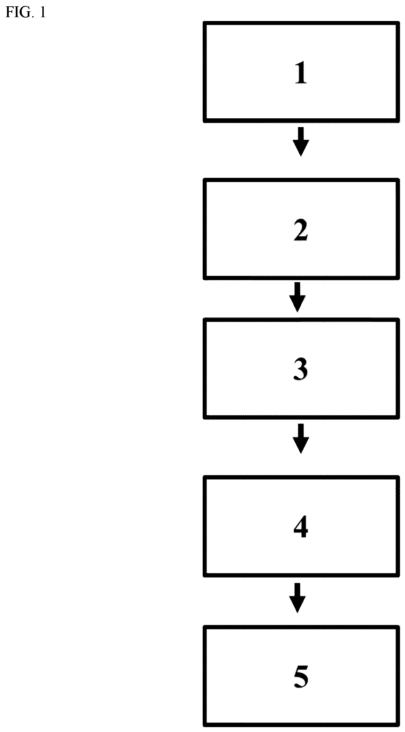

Fig. 1 is a schematic representation of the method described herein comprising the following steps (1) providing a cell culture fluid sample comprising mammalian cells expressing a protein of interest at a concentration within the range of between 0.2g/l to 10 g/l (2) Filtering said cell culture fluid sample over at least one filter (3) Displacing O₂ in the filtered cell culture fluid sample (3) Collecting at least one sample of the O₂-displaced filtered cell culture fluid sample (4) Separating the proteins in said at least one O₂-displaced filtered cell culture fluid sample according to their size under non-denaturing conditions(5) Determining the disulfide bond reduction potential of the protein of interest.

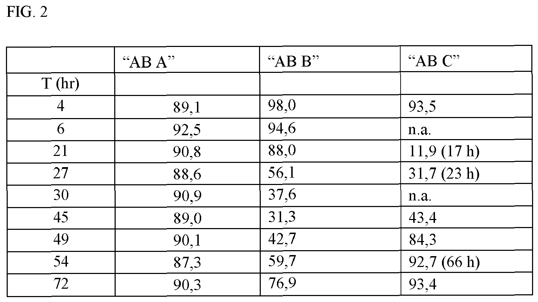

Fig. 2 depicts the results of the method for assessing disulfide bond reduction potential as described herein for three different monoclonal antibodies. Antibody A "AB A" showed almost no reduction in method for assessing disulfide bond reduction as demonstrated by the high percentage of intact antibody in all samples over the time course of the experiment. Antibody B "AB B" exhibited some reduction, leading to a level of intact antibody of 30 - 40% after 30 - 48 hr. Antibody C "AB C" showed the largest reduction potential with only very little intact antibody present in the sample after 12-24 hr. All samples were processed using a throughput of 70 LMH on a single stage dual-layer depth filter of 1 - 25 µm pore size. It should be noted that for AB C similar results were obtained from a cell culture processed according to the method described herein, but originally cultured on 200L scale. Hence, the size/volume of the cell culture does not seem to matter for the success of the method described herein, but rather the method described herein manages to reflect the manufacturing scale processing and purification conditions very adequately. Hence if the protein of interest is susceptible to disulfide bond reduction under large scale processing and purification conditions, which also include back pressures etc. not seen on small scale, said protein of interest is also susceptible to disulfide bond reduction in the method described herein.

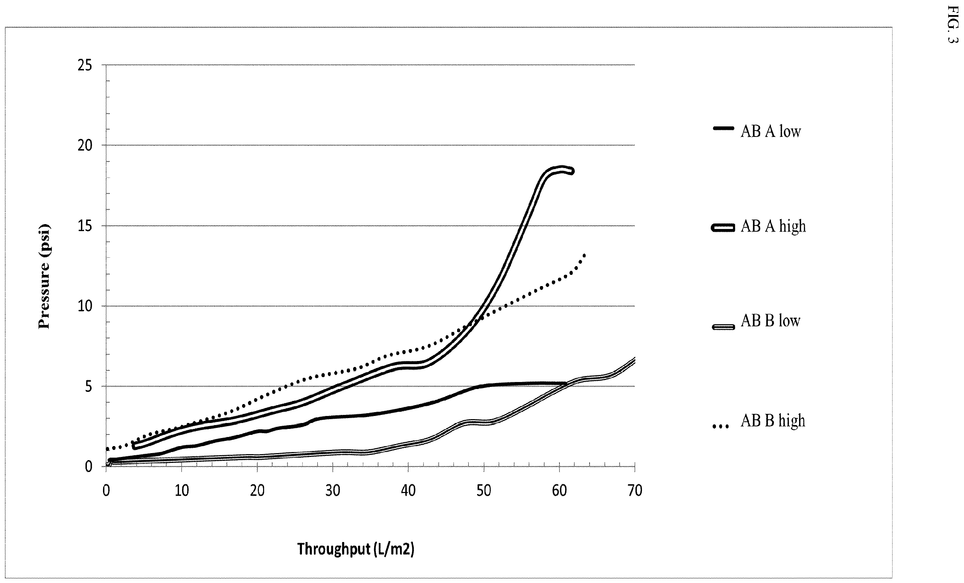

Fig 3 depicts that via varying the differential pressure applied to a filter the throughput also varies. Shown are the results for the same culture of two different monoclonal antibodies here Antibody A "AB A" and Antibody B "AB B" where each antibody was filtered once at high flux between 55 -75 LHM and once at low flux between 27-30 LMH through a single stage dual-layer depth filter of 1 - 25 µm pore size. Moreover, the specific throughput at a specific pressure differs for different proteins of interest thus extensive preliminary experiments are required to identify the optimal filter for a filtration of a given protein or interest on large scale. The pressure values are given in psi (pound-force per square inch) where 1 psi ~ 0,069 bar.

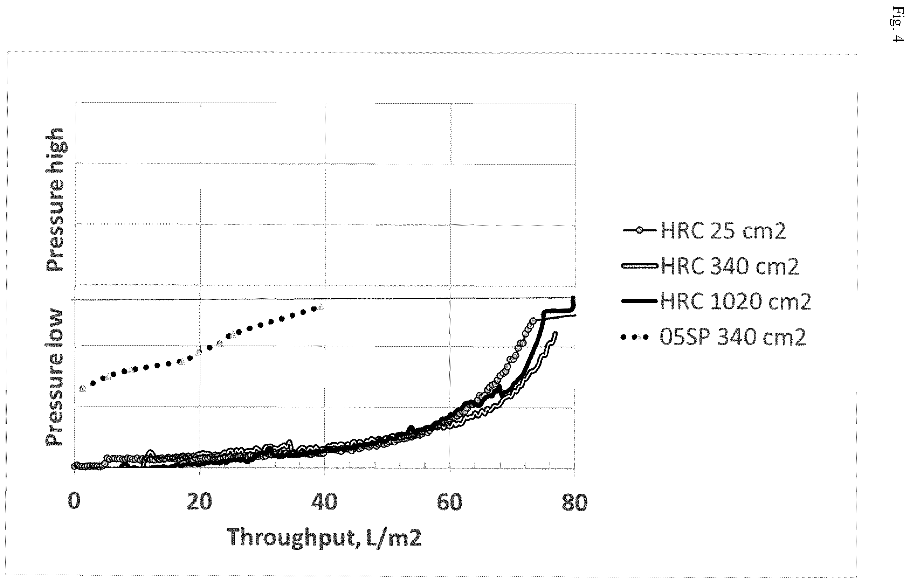

Fig 4 demonstrates that Harvest RC filters of different sizes showed similar delta pressure (dP) profiles to reach a certain throughput. Hence, time-consuming and tedious scale up experiments can be omitted. In the shown example, cell culture fluid aliquots from one same batch were filtered with varying sizes (25 - 1020 cm²) of charged non-woven filters and the resulting back-pressure was low. Throughputs of around 70 l/m² were obtained at low pressure end-points, here 5 psi=0.34 bar. It is shown that to reach a similar throughput with a single stage dual-layer depth filter of 1 - 25 µm pore size a higher differential pressure was required. Hence the charged non-woven filter allowed for a higher throughput at a lower pressure thereby being gentler on the cells while still allowing for an efficient process on large scale.

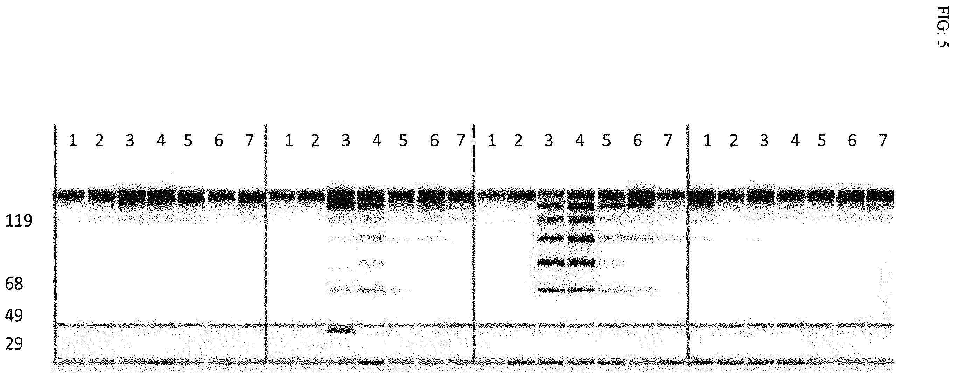

Fig. 5 shows a result of a CALIPER Gel analysis after carrying out the method for assessing disulfide bond reduction potential of a protein of interest, here a monoclonal antibody termed "AB D" . In detail a cell culture fluid expression a protein of interest, here a monoclonal antibody, was split into four fractions. One was filtered through a single stage dual-layer depth filter of 1 - 25 µm pore size at a target rate of 30 LMH (the results are shown on the far left lanes 1-7 in the gel), one fraction was filtered through the same type of single stage dual-layer depth filter at target rate of 50 LMH (the results are shown in the middle left lanes 1-7 in the gel), and one fraction was filtered through the same type of single stage dual-layer depth filter at target rate of 70 LMH (the results are shown on the middle right lanes 1-7 in the gel), the last fraction was filtered through a charged non-woven filter (the results are shown on the right lanes 1-7 in the gel). Lanes 1 represented un-treated filtered samples while lanes 2 - 7 represent samples drawn following O₂ displacement at 0, 24, 48, 72, 96, and in this case exceptionally 168 hours. The values given on the y axis refer to a standard protein size marker. In this example the assessed protein of interest showed disulfide bond reduction from a target flux of 50 LMH after filtering through the single stage dual-layer depth filter of 1 - 25 µm pore size. However, no reduction was observed with a charged non-woven filter (at 100 LMH flux). Hence using the method described herein one can determine whether appropriate mitigation methods should be implemented already after small scale experiments and whether using a charged non-woven filter would also be a suitable mitigation method.

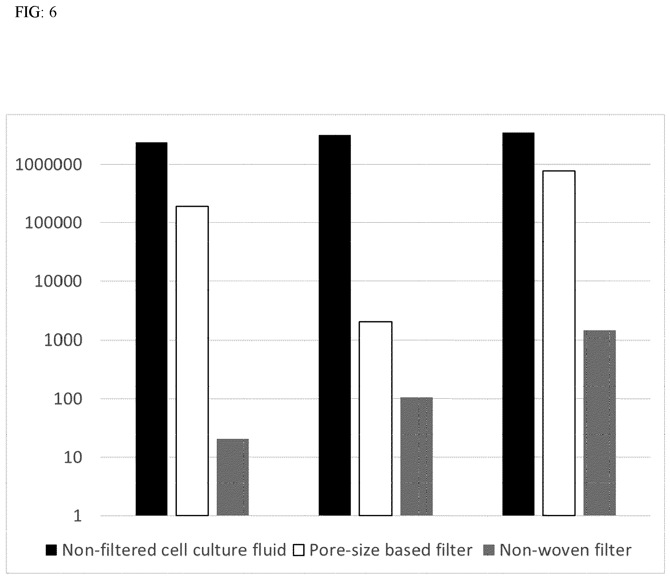

FIG 6 shows host cell DNA in parts per billion (ppb) before (dark bars) and after filtration of fractions of the same cell culture fluid with a two-stage depth filter system of 0.1 - 10 µm pore size (white bars) or a charged non-woven filter (gray bars). The host cells in this example were Chinese hamster ovary cells. The results demonstrate that the charged non-woven filter retains more of the host cell DNA than the two-stage depth filter system of 0.1 - 10 µm pore size.

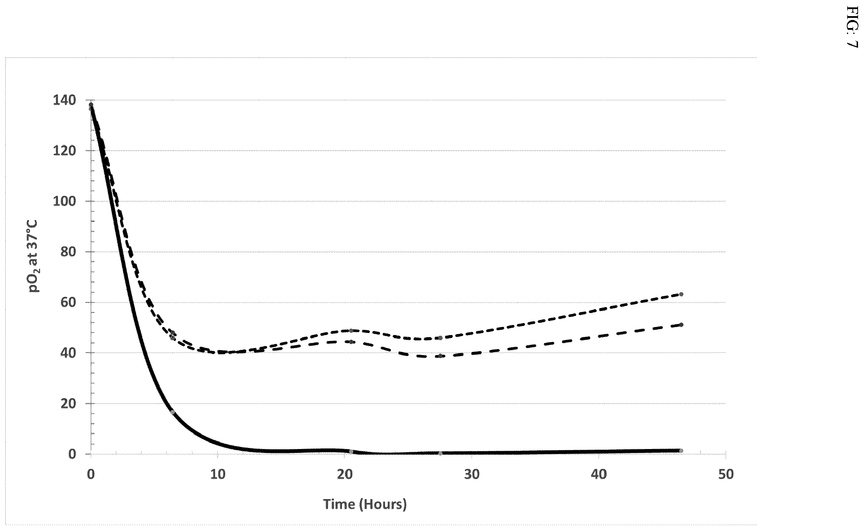

Fig. 7 depicts that the oxygen level in a cell culture samples comprising a monoclonal antibody "AB E" filtered through a single stage dual-layer depth filter of 1 - 25 µm pore size decreases to almost zero (black solid line) whereas filtration over a charged non-woven filter at low (dotted line) and moderate (dashed/broken line) pressure did not decrease the oxygen content in the filtered cell culture sample to a similar extent.

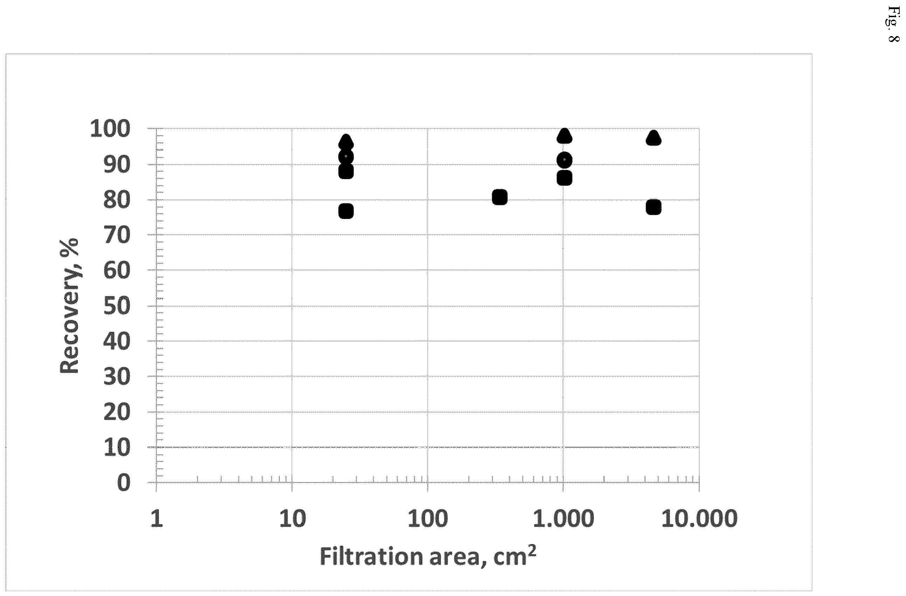

Fig. 8 depicts that the recovery of three different monoclonal antibodies - "AB A" (triangles) "AB F" (circles) "AB G " (squares) - across varying filter area of charged non-woven filters is consistent.

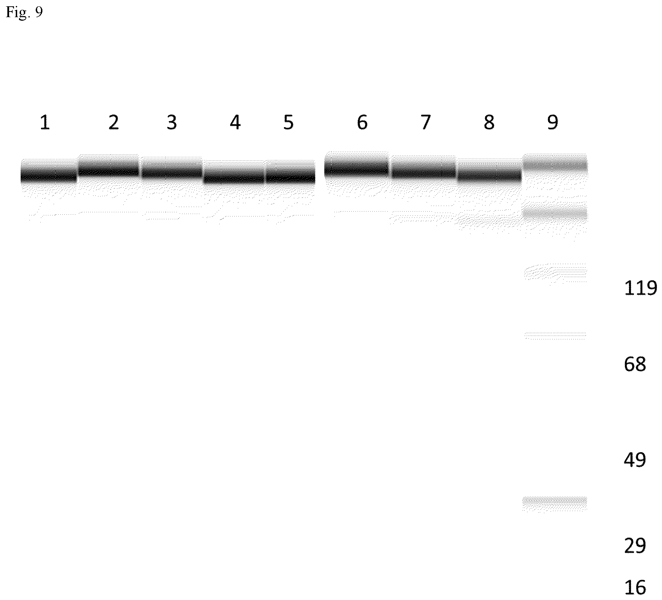

Fig. 9 shows a result of a CALIPER Gel analysis after carrying out the method for assessing disulfide bond reduction potential of a protein of interest, here monoclonal antibody "AB E" . In detail a cell culture fluid expressing AB was split into two fractions. The first fraction was filtered through a charged non-woven filter to a setpoint dP (at low pressure, here 5 psi=0.34 bar) but not further blown down. The second fraction, on the other hand, was blown down dry after the filtration (lanes 6-9). Lane 1 depicts a sample of the cell culture fluid prior to filtration, lanes 2-5 depict samples of the filtered but not blown down fraction, where the samples were taken at 24, 48, 72 and 96 hours after O₂ displacement. Lanes 6-9 depict samples of the filtered and blown down fraction, where the samples were taken at 24, 48, 72 and 96 hours after O₂ displacement.

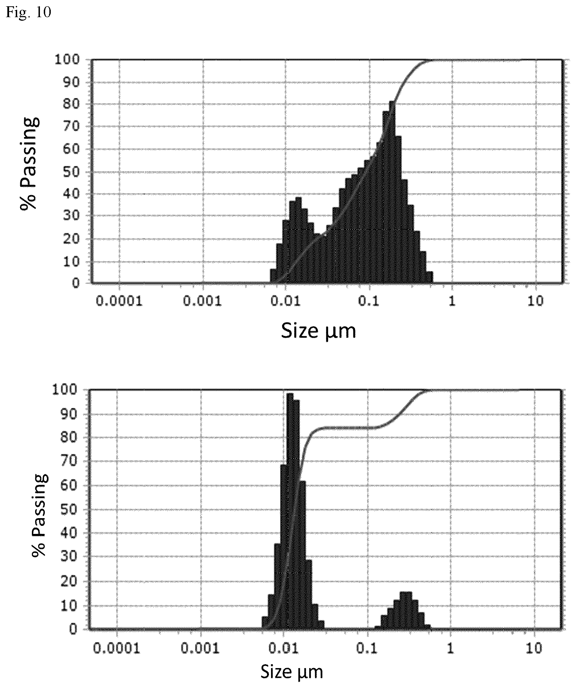

Fig. 10 depicts the results of a particle size analysis carried out with the Dynamic Light Scattering (DLS) device Nanotrac Flex from Microtrac. It is shown that after filtration of two fractions of the same cell culture sample comprising a protein of interest - here "AB D" -over a dual-stage depth filter with a pore size of between 0,1µm and 10µm (top diagram) considerably more of large particles are present than after filtration over a charged non-woven filter (bottom diagram). In detail the graphs show % abundance of particles ("% passing) vs. particle size. Moreover, the left hand side peak (centered around 0.01 mm on the bottom diagram) corresponds to the protein of interest. Hence after just one stage of filtration over a charged non-woven filter the protein of interest has been enriched.

### DETAILED DESCRIPTION

What is described herein relates to a method for assessing disulfide bond reduction potential of a protein of interest.

Whenever appropriate, terms used in the singular will also include the plural and vice versa. In the event that any definition set forth below conflicts with the usage of that word in any other document, including any document incorporated herein by reference, the definition set forth below shall always control for purposes of interpreting this specification and its associated claims unless a contrary meaning is clearly intended (for example in the document where the term is originally used). The use of "or" means "and/or" unless stated otherwise. The use of "a" herein means "one or more" unless stated otherwise or where the use of "one or more" is clearly inappropriate. The use of "comprise," "comprises," "comprising," "include,"
"includes," and "including" are interchangeable and are not limiting. For example, the term "including" shall mean "including, but not limited to."

What is described herein relates to a method for assessing disulfide bond reduction potential of a protein of interest comprising the following steps:
- Providing a cell culture fluid sample comprising mammalian cells expressing a protein of interest at a concentration within the range of between 0.2 g/l to 10 g/l
- Filtering said cell culture fluid sample over at least one filter
- Displacing O₂ in the clarified cell culture fluid sample
- Collecting at least one sample of the O₂-displaced clarified cell culture fluid sample
- Separating the proteins in said at least one O₂-displaced, filtered cell culture fluid sample according to their size under non-denaturing conditions
- Determining the disulfide bond reduction potential of the protein of interest.

Using this method, it has become feasible to determine at a small - e.g. bench size - scale whether a given protein of interest is susceptible to disulfide bond reduction during purification at a larger - *e.g.* manufacturing - scale. So far the phenomenon of disulfide reduction of a protein of interest e.g. an antibody has often been observed only at large scale, leading to a difficulty of predicting the likelihood of disulfide bond reduction using small-scale experiments. Thus, using the method described herein allows an early assessment for a given protein of interest whether that protein of interest is susceptible to disulfide bond reduction. Hence the method enables a faster, less tedious and more cost-efficient process development, since mitigation measures against disulfide bond reduction can be implemented and integrated into a production and purification process early on, if necessary, instead of having to adapt or re-work a production and purification process established at small- and pilot-scale (*e.g.* 2 - 50 L) when disulfide bond reduction is observed for the first time at larger *e.g.* 200 - 2000 L manufacturing scale.

So far disulfide reduction events have been, for example, mimicked on small scale by purposefully lysing cells following cell culture through homogenization. Experiments were conducted using the lysed cell material which was optionally filtered and centrifuged prior to the experiments. Alternatively, the lysed cells were blended with the non-purposefully lysed cells of the same original cell culture in order to obtain final cell culture pools with desired target amounts of total lysis levels (Trexler-Schmidt *et al.* 2010). However, the homogenization in this setting takes the cells to extreme lysis and then dilutes them back to a particular percentage compared to the manufacturing process where (unwanted) mechanical cell lysis from the harvest operation is added to the starting level of lysis at the end of the cell culture process (Trexler-Schmidt *et al.* 2010). Hence this cell-lysis-by-homogenization model is able to mimic conditions in a cell culture fluid comprising lysed cells but does not adequately reflect the operational settings of a manufacturing process.

As used herein the term `bench scale' refers to a cell culture volume in the range of between 1 ml to 50 1 whereas the term `large scale' refers to a cell culture volume in the range of 50 to 5000 1.

It was found that of different protein of interest cultures generated different backpressure and oxygen profiles during and after filtration on bench-scale, respectively.

In one embodiment of the method described herein the protein of interest is an antibody, a monoclonal antibody, a bi- and tri-specific antibody, a multi-specific antibody, another antibody variation where the two or more chain components are held together by inter-chain disulfide bond(s).

In one embodiment of the method described herein, the density of the mammalian cells expressing the protein of interest in the cell culture fluid sample is in the range of between 5 × 10⁶ cells/mL to 30 × 10⁶ cells /ml.

In one embodiment of the method described herein the mammalian cells are selected from the group comprising Chinese Hamster Ovary (CHO) cells, NS0 murine myeloma cells, PER.C6 human cells and HEK293 cells.

In one embodiment of the method described herein the at least one provided cell fluid sample has a volume in the range of between 10 - 2000 ml. In another embodiment of the method described herein the at least one provided cell fluid sample has a volume in the range of between 200 - 250 ml.

In one embodiment of the method described herein, the step of separating of the proteins in said at least one O₂-displaced filtered cell culture fluid sample according to their size i.e. their molecular weight is carried out using a gel electrophoresis.

Gel electrophoresis is a method for separation and analysis of macromolecules (e.g. DNA, RNA and proteins) and their fragments, based on their size and charge. Under an application of electric field, molecules can migrate through a gel made of agarose or polyacrylamide. The electric field consists of a negative charge at one end which pushes the molecules through the gel, and a positive charge at the other end that pulls the molecules through the gel. The molecules being sorted are dispensed into a well in the gel material. The gel is placed in an electrophoresis chamber, which is then connected to a power source. When the electric field is applied, the larger molecules migrate more slowly through the gel while the smaller molecules migrate faster. The different sized molecules form distinct bands on the gel. The gel *i.e.* the matrix is a cross-linked polymer whose composition and porosity are chosen based on the specific molecular weight and composition of the target to be analyzed. If several samples are loaded into adjacent wells in the gel, they will run parallel in individual lanes. Depending on the number of different molecules, each lane shows the separation of the components from the original mixture as one or more distinct bands, one band per component. Incomplete separation of the components can lead to overlapping bands, or indistinguishable smears representing multiple unresolved components. Bands in different lanes that end up at the same distance from the top contain molecules that passed through the gel at the same speed, which usually means they are approximately the same size. There are molecular weight size markers available that contain a mixture of molecules of known sizes. If such a marker was run on one lane in the gel parallel to the unknown samples, the bands observed can be compared to those of the unknown to determine their size.

Exemplary devices for analyzing samples in a conventional gel electrophoresis are BioRad or LifeTech gel systems.

In one embodiment of the method described herein, the step of separating the proteins in said at least one O₂-displaced, filtered cell culture fluid sample according to their size i.e. their molecular weight is carried out using a capillary gel electrophoresis

Capillary Gel Electrophoresis (CGE) is a type of gel electrophoresis where the gel is comprised in capillaries which provide a large ratio of surface area to volume than slab gels.

In one embodiment of the method described herein the capillary gel electrophoresis is performed using a Caliper lab chip LabChip GX II, BioAnalyzer chip or Maurice CE-SDS system. Sample preparation is carried out according to the manufacturer's instructions. Digital gel-like images are generated using the LabChip GX software.

In one embodiment of the method described herein the step of determining the disulfide bond reduction potential further comprises separating the proteins in said at least one O₂-displaced, filtered cell culture fluid sample according to their size under denaturing conditions.

This protein separation according to molecular weight under denaturing conditions allows an assessment whether different parts of the protein of interest which are held together by disulfide bonds - *e.g.* the heavy and lights chains of an antibody - are expressed in the correct ratio by the cell culture. Hence, if the ratio is correct the assessment allows for the conclusion that any non-intact form of the protein of interest observed after separating the proteins in said at least one O₂-displaced, filtered cell culture fluid sample according to their size under non-denaturing condition is due to disulfide bond reduction.

In one embodiment of the method described herein prior to separating the proteins in said at least one O₂-displaced, filtered cell culture fluid sample according to their molecular weight size under denaturing and/or non-denaturing conditions the proteins in said at least one O₂-displaced, filtered cell culture fluid sample are purified on affinity resin(s) or via ionic exchange chromatography.

By purifying the proteins in said at least one O₂-displaced, filtered cell culture fluid sample affinity resin(s) or via ionic exchange chromatography, determining the disulfide bond reduction potential of the protein of interest becomes easier since the protein of interest is enriched. Moreover, the purification step removes a variety of contaminants such as host cell proteins in a non-purified O₂-displaced, filtered cell culture fluid sample. Hence purified O₂-displaced, filtered cell culture fluid samples allow for an easier protein concentration determination. Thus, equal amounts of antibodies collected at various timepoints can be loaded for gel electrophoresis analysis.

An exemplary method for determination of protein concentration is *e.g.* by Abs₂₈₀. The method makes use of the fact that proteins in solution absorb ultraviolet light with absorbance maxima at 280 and 200 nm, and that the relationship of absorbance to protein concentration is linear. For calculation of protein concentration, the Beer-Lambert equation can be used: Abs = log (I/I₀) = εlc, where *Abs* is the absorbance at 280 nm, *I* is the intensity, *ε* is the molar extinction coefficient, *l* is the optical path length in cm and c is the concentration of the solution in mol/dm³. In order to perform the determination a spectrophotometer with UV lamp and a quartz cuvette are required. Hence the method is fast and convenient, since no additional reagents or incubations are required, no protein standard needs to be prepared and the procedure does not consume the protein. Alternatively, ProA-HPLC can be used to determine protein e.g. antibody concentration.

In one embodiment of the method described herein the affinity resin(s) is chosen from as the group consisting of Protein A, Protein G, Protein L and any synthetic or peptides that specifically bind to an epitope on an antibody or its derivatives. In one embodiment of the method described herein the ionic exchange chromatography is chosen from anion exchange, cation exchange and/or mixed mode chromatography. Other chromatography techniques such as hydrophobic interactions may also be used. To a skilled person it is clear which method of purification to use in which scenario. For example, if an affinity chromatography is available which matches the protein of interest this is usually chosen over ion exchange resins. However, the conditions under which the purification is performed - e.g. bind-and-elute, flow-through or mixed-mode - also have an influence on the choice of the skilled person for the most suitable purification method.

In one embodiment of the method herein, at least one other sample in addition to the O₂-displaced filtered cell culture fluid sample is collected. Said at least one other sample is collected after filtering said cell culture fluid sample over at least one filter. In one embodiment a sample of the filtered cell culture fluid sample is taken every 5 minutes until all of the cell culture fluid sample was filtered over at least one filter.

In one embodiment of the method described herein said at least one other sample is collected after filtering said cell culture fluid sample over at least one filter wherein said at least one sample collected following filtration is assessed for turbidity, Dynamic Light Scattering (DLS) for protein size measurement, and O₂ level by Blood Gas Analysis (BGA).

In one embodiment of the method described herein displacing O₂ in the filtered cell culture fluid sample is carried out until the O₂ in the filtered cell culture fluid sample is lower than 40 mmHg at 37 °C. In one embodiment method described herein displacing O₂ in the filtered cell culture fluid sample is carried out using N₂.

In one embodiment of the method described herein more than one sample of the O₂ displaced filtered cell culture fluid sample is collected. In one embodiment of the method described herein at least one sample of the O₂-displaced filtered cell culture fluid sample is collected as soon as O₂ level is lower than 40 mmHg and further samples are collected at least once a day for at least 72 hours. In one embodiment, 2-3 samples are collected at different time points every day for 120 hrs.

In one embodiment of the method described herein the step of determining the disulfide bond reduction potential of the protein of interest comprises calculating the percentage of the intact form of the protein of interest for the at least one sample of the O₂-displaced filtered cell culture fluid sample.

The terms native, non-reduced and non-denaturing as well as denaturing and reduced are used synonymously herein.

To a skilled person it is clear that also the percentage of the non-intact form of a given protein of interest can be calculated in order to determine the disulfide bond reduction potential of the protein of interest since both values are based on the same assumption *i.e.* that any non-intact form of a protein of interest is due to disulfide bond reduction.

If this method of determining the disulfide bond reduction potential of the protein of interest is chosen, then at least one sample of the O₂-displaced filtered cell culture fluid sample is collected between 24 hours and 72 hours after filtering the cell culture fluid sample over at least one filter. Should this at least one sample of the O₂-displaced filtered cell culture fluid sample show intact protein of interest in the range of 70 % to 0%, the protein of interest is likely to exhibit disulfide bond reduction potential at large scale *(cf.* Table 1 and Table 2 below).

**Table 1 Intactness of AB B on large scale (in this example 200 liters)**

| | AB B From cell culture | Positive control |
|---|---|---|
| Intact (% total) | 4.1% | 83.7% |

In addition to the percentage of intact protein of interest of AB B as given in Table 1 it was also assessed whether light and heavy chains of AB B were produced by the cell culture in the correct ratio (data not shown). As this was the case the result as given in Table 1 demonstrated that the monoclonal antibody AB B exhibited reduction during purification on large scale. This reduction was also seen on small scale when employing the method for assessing disulfide bond reduction potential of a protein of interest as described herein (cf. Table 2)

**Table 2 Intactness of AB B on small scale (in this example 2 liters, corresponding to AB B of Fig. 2)**

| Time after O₂ displacement | AB B % of intactness |
|---|---|
| 6 | 94,6 |
| 21 | 88,0 |
| 27 | 56,1 |
| 30 | 37,6 |
| 45 | 31,3 |
| 49 | 42,7 |

In one embodiment determining the disulfide bond reduction potential of the protein of interest comprises calculating the percentage of the intact form of the protein of interest for more than one sample of the O₂-displaced filtered cell culture fluid sample. In one example of this embodiment O₂-displaced filtered cell culture fluid sample are taken at regular intervals e.g. within 18 hours to 72 hours, after filtering the cell culture fluid sample over at least one filter and calculating the percentage of the intact form of the protein of interest is done for all O₂-displaced filtered cell culture fluid samples. An example of such an embodiment can be found in Fig. 2.

In one embodiment of the method described herein, the step of determining the disulfide bond reduction potential further comprises a comparison of the percentage of intact form of the protein of interest or the percentage of the non-intact form of the protein of interest or both to a reference protein in order to assess the relative disulfide bond reduction potential of the protein of interest.

When this approach of determining the disulfide bond reduction potential of the protein of interest is chosen, a reference protein which is known to show disulfide bond reduction using the method described herein is used to compare the percentage of intact form of the protein of interest or the percentage of the non-intact form of the protein of interest or both. If a lower percentage of the intact form of the protein of interest is present than of the reference protein, then the protein of interest is deemed to exhibit disulfide bond reduction potential on large scale (*cf.* data presented in Table 1 and Table 2 and Fig. 2).

In one embodiment of the method described herein the filtration is carried out with a single-stage dual-layer depth filter of 1 - 25 µm pore size, or a two-stage depth filter of 0.1 - 10 µm pore size range or a charged non-woven filter with a membrane area in the range of between 0.004 and 0.500 m².

Depth filters are the variety of filters that use a porous filtration medium to retain particles throughout the medium, rather than just on the surface of the medium. These filters are commonly used when the fluid to be filtered contains a high load of particles because, relative to membrane filters, they can retain a large mass of particles before becoming clogged. Depth filters often comprise a fibrous network that is typically made up of cellulose fibers with nominal pore size range as well as filter aids capturing particulates - thereby increases filtration throughput - and optionally polymeric binders with variable levels of ionic charges. In a single stage (also termed one-stage) dual layer depth filter with 1-25 µm pore size two layers comprising at least cellulose fibers with nominal pore size range as well as filter aids exist which differ in their nominal pore size. The cells and other components of a cell culture fluid sample travel the length of the depth filter until they are retained/trapped in section(s) of the depth filter of limiting pore size. Usually, a single stage (one stage) depth filtration refers to the use of a single type of filter containing two discontinuous ranges of pore sizes in the range of 25 µm to 0.4 µm within a filter. Examples of a single-stage depth filtration system are PDG4 and PDK5 from Pall (Port Washington, New York, USA). A 2-stage depth filtration set-up typically refers to two different depth filters hooked up in series, with each stage of the depth filter having different pore sizes. Example of the 2-stage filtration systems are the ZetaPlus filters with the first stage filters having pore sizes of 0.1 - 10 µm (3M), and the second stage filters having pore sizes of 0.1 - 3 µm thus, an overall pore size range between 0.1- 10 µm. Other examples of two-stage filtration systems are Clarisolve in combination with Millistack from Millipore Sigma (Burlington, MA, USA). A three-stage filtration system is typically a two-stage system having a third membrane such as 3M Emphaze AEX Hybrid Purifier.
Charged non-woven filters such as the Harvest RC filter (3M, Maplewood, MN, USA) do not function by pore size mechanism. They comprise a non-woven fibrous network that supports a defined number of functionalized ligands along these networks. The functionalized ligands are positively charged, and thus will interact with, bind to, and retain cell culture fluid components that are negatively charged such as intact CHO cells, cell fragments, cellular debris, DNA and CHO host cell proteins (HCPs). The intact cells are retained by the functionalized ligands due to the negatively charge of their phospholipid moieties of cell membrane.

It was found that in cases of proteins of interest e.g. monoclonal antibodies with low isoelectric points (pI) adsorption and filtration profiles on a charged non-woven filters such as the Harvest RC filter differ. While in one case the monoclonal antibody adsorbed to the anionic charged filter a second monoclonal antibody did not. A strategy to clarify these different acidic pI proteins of interest was devised.

In general, an accurate estimation of the optimum (depth) filter size *i.e.* of the filter area in m² is critical to the overall purification of a protein of interest from a cell culture fluid. If the chosen depth filter is too large the process will not be cost-effective. In contrast, if the chosen depth filter area is too small, then operational challenges such as blockage of the filter and a higher level of process-related impurities breaking through the filter may arise. However precise estimates on required filter size can be complicated since a variety of parameters such as the flux of filtration, total cell density and viability of cell culture, level of product aggregates and miscellaneous cell culture conditions can influence the behavior of the cell culture fluid during filtration (*cf.* Goldrick *et al.* 2017).

The *term flux* refers to how fast a fluid (*e.g.* a cell culture fluid sample) is pumped wherein the value is normalized to each m² of the respective filter. Its unit is L of cell culture fluid sample per hour per m². It is regarded as input for a filtration.

The term *throughput* describes the volume of a filtrate and hence a filtration output. For example for a particular flux *e.g.* 70 L/hr for 1m² filter, the throughout could be 40 L of a cell culture fluid sample per 1 m² of filter at a delta pressure of 0.68 bar (10 psi) and 60 L of the cell culture fluid sample per 1 m² of filter at a delta pressure of 1.03 bar (15 psi). Hence in this case, the throughput of the filter is 40 L/m² and 60 L/m² at dP = 0.68 and dP = 1.03 bar, respectively. In other words, a higher throughput of a filter is possible at the expense of the higher differential pressure

At a lower flux *e.g.* 25 L/hr for 1m² filter for the same filter the throughput at the respective delta pressures of 0.68 and 1.03 bar will typically be higher. However, if the flux is low, then processing a cell culture fluid sample would take considerably longer time.

Additionally, since depth filter components such as diatomaceous earth are derived from natural, nonrenewable sources, there can be quality- and performance-variations from different batches of depth filters over time. Thus, numerous time-consuming filtration trials are often performed for a given protein of interest expressed in a given cell line under the chosen conditions in order to find the optimal depth filter size.

High differential pressure (ΔP) across a filter -- *e.g.* more than 0.55 bar (8 psi) of the primary depth filter during harvest -- has been identified as a key operational parameter that contributes to cell lysis (O'Mara *et al* 2019). Hence the differential pressure at which a cell culture fluid is filtered through the respective (depth) filter should be controlled in order to prevent or minimize cell lysis. However, filtering below 0.55 bar (8 psi) DP is not always an economic option. This is the case as a larger filter area (m²) which would in principle allow for filtering a given cell culture fluid at a lower differential pressure while still allowing for sufficient throughput is unlikely to meet process economic criteria.

The intrinsically low differential pressure - at which charged non-woven filters can be operated while still achieving a high throughput at high cell densities - has been demonstrated to cause only minimal cell shear (see 3M brochure "Harvest RC single-stage chromatographic purification for recombinant protein therapeutic manufacturing 2021" as well as Fig. 3) and thus is unlikely to have significant cell lysis. As such, cell culture clarified by synthetic non-woven fibrous filters grafted with functionalized ligands can be expected to have a lower likelihood/ potential of disulfide reduction. Moreover, even at moderate pressure filtration over a charged non-woven filter results in less oxygen depletion compared to filtration over a single stage dual-layer depth filter of 1 - 25 µm pore size (*cf.* Fig. 7).

Synthetic charged non-woven filters also facilitate a more predictable and consistent scale-up filtration. For a given cell culture density as determined by packed cell volumes (PCV) robust reproducibility and performance across various filtration areas of the charged filters was achieved. As shown in Fig. 3 the throughput of three different filter areas/units of the charged filter were around 75 L per m2 of filter at a low pressure of here DP = 0.34 bar (5 psi) end-points. Similarly, the recovery of the products across varying filter areas/units were also consistent *(cf.* Fig. 8). Hence, time-consuming and expensive trials for matching filter size to the clarification process in question can be shortened/abbreviated, thereby further simplifying process development.

Another step that can be simplified with charged non-woven filter is flushing of the filter prior to use. This is the case since if a charged non-woven filter is used only one flush has to be performed as compared to sequential flushes with Water for Injection (WFI) *i.e.* medical-grade water and phosphate buffered saline (PBS) typically performed with a 2- or 3-stage filtration system *i.e.* one flush per stage. To a skilled person it is immediately apparent that the simplification related to filter flushing multiplies with scale.

For example, if a two-stage depth filter of 0.1 - 10 µm pores - *e.g.* a ZetaPlus 05SP01- is to be used with a throughput of a cell culture fluid of 70 L/m² and a typical 2: 1: 1 ratio in a 3-stage filtration system, one may end up with 18:9:9 capsules. This would be the case if the total filtration area required for 2000 L of cell culture fluid would be in the range of 28.5 m² at the first stage, 14.3 m² each for the second and third stage filters. As a capsule has 1.6 m² of filtration area this would result - 2000/70/1.6 = 17.9 capsules - *i.e.* in 18 capsules for the first stage. Consequently, if a 2:1:1 ratio is to be used the relation would be 18:9:9 capsules for the first, second and third stage filters, respectively. If a filter holder can fit 7 capsules, the first stage filters of 18 capsules are assembled into 3 filters holders. Similarly, the second stage filter capsules are assembled into 2 filter holders, and third stage filters are assembled into 2 filter holders. Hence in this example in total 7 filter holders are used. In this exemplary case according to the filters' manufacturers typical instructions each of the first two stage filters (in 3 + 2 = 5 holders) needs to be flushed with WFI. The third stage needs to be flushed with PBS before it is connected with the first and second stage filters. The entire 3-stage filter assembly is then flushed and equilibrated with PBS before the filter assembly is used to filter cell culture fluid. In contrast, the same cell culture fluid can be filtered with charged non-woven filters. In this case 18 charged non-woven filters would be fitted into 3 filter holders. They can be flushed with minimal or no WFI before use. Hence, in addition to requiring fewer man-hours for assembling the filter holders and flushing the filters, less WFI and PBS buffer volume are required. The charged non-woven filtration system also takes up a much smaller footprint, and as fewer parts are to be assembled, its use is less error prone.

Furthermore, (single-stage) charged non-woven filters facilitate downstream processing *i.e.* purification of a protein of interest. As shown in Fig 6, the amount of DNA impurity is considerably lowered by the charged non-woven Harvest RC filter compared to a filter system relying on pore size. The lower DNA content in the cell culture filtered by the charged non-woven filter thus leads to a higher ProA binding capacity, a longer resin lifetime and likely an absence of precipitation of Protein A eluate following an upward pH adjustment of Viral Inactivated Protein A eluate, thereby facilitating a downstream processing free of unexpected operational disruptions.

Furthermore, it was found that a charged non-woven filter in a single step not only enriched the 0.01 nm pool of protein of interest but also removed more of contaminant host cellular components larger than ~0.05 nm *(cf.* Fig. 10). In comparison, the pore size based depth filters were able to enrich for the 0.01 nm product pool but a significant pool of larger size cellular component contaminants *(cf.* Fig. 10) remained in the sample after filtration.

In addition, employing a charged non-woven filter means that overall less membrane area is used compared to a single-stage dual-layer depth filter of 1 - 25 µm pore size, or a two-stage depth filter of 0.1 - 10 µm pore size range. Hence also less product is retained on a charged non-woven filter, which in case of single-stage dual-layer depth filters of 1 - 25 µm pore size, or a two-stage depth filters of 0.1 - 10 µm pore size might be recovered e.g. via filtration chase and blow-down of the filter train to achieve economically acceptable yields.

Furthermore charged non-woven filters can be employed in intensified manufacturing systems.

Moreover, it was found that cell culture fluid processed with a charged non-woven filters such as Harvest RC filter (3M, Maplewood, MN, USA) contained fewer retrovirus-like particles compared to cell culture fluid processed with a single-stage dual-layer depth filter of 1 - 25 µm pore size, or a two-stage depth filter of 0.1 - 10 µm pore size range (cf. Fig. 11- Fig. ). In one example the reduction was 2 *log* i.e. the cell culture fluid clarified via a charged non-woven filters - here a Harvest RC filter (3M, Maplewood, MN, USA) - contained 10⁴ RVLP whereas the same cell culture fluid clarified via a single-stage dual-layer depth filter of 1 - 25 µm pore size, or a two-stage depth filter of 0.1 - 10 µm pore size range - here a ZetaPlus and Emphaze AEX Hybrid Purifier (ZP+HP) respectively -contained 10x⁶ RVLP. The lower concentration of particles (RVLP) per mL in cell culture clarified using the charged non-woven filter lowers the overall putative RVLP to be cleared from the clarified cell culture fluid during subsequent purification of the protein of interest. Hence employing the charged non-woven filter benefits the viral clearance safety margin of the purification process i.e. the manufacturing process of the protein of interest and facilitates meeting viral safety margins. Thus, clarification of cell culture has impacts on the overall safety margin of viral clearance capacity of a manufacturing process. Thus employing charged non-woven filters leads to an overall more efficient process for enriching a protein of interest, which is beneficial for further downstream purification steps since fewer contaminants have to be removed.

In one embodiment of the method described herein if a two stage depth filter of 0.1 - 10 µm pore size is used the flux is set to 100 LMH (liters /m² per hour).

In one embodiment of the method described herein if a single-stage dual-layer depth filter is used the flux is set to at least 70 LMH (liters /m² per hour). It has been shown that using this setting it is most likely to find disulfide bond reduction of a protein of interest possibly because this flux most adequately reflects the back pressure experienced by a cell culture fluid on large scale filtration. Hence it is feasible to determine the disulfide bond reduction potential of the protein of interest via calculating the percentage of the intact form of the protein of interest or the percentage of the non-intact form of the protein of interest or calculating both. In addition, it has been shown that if this approach is used in a small scale setting the observed disulfide bond reduction corresponds even better to disulfide bond reduction on large scale. This is demonstrated in Table 2a below. Table 2a compares the values of intactness percentage of Table 2 above, which were derived using 70 LMH filtration, with the intactness percentage at 30 LMH *(cf.* Table 1 and Table 2a). It can be seen that the flux of 70 LMH mimics the situation on large scale *(cf.* Table 1) better than that at 30 LMH flux.

**Table 2a small scale (corresponding to AB B of Fig. 2 and Table 2)**

| Time after O₂ displacement | AB B % of intactness (70 LMH) | AB B % of intactness (30 LMH) |
|---|---|---|
| 6 | 94,6 | 97,5 |
| 21 | 88,0 | 98,1 |
| 27 | 56,1 | 95,8 |
| 30 | 37,6 | 96,3 |
| 45 | 31,3 | 98,0 |
| 49 | 42,7 | 97,3 |

In one embodiment of the method described herein if no reduction is observed with the charged non-woven filter with a membrane area in the range of between 0.004 and 0.500 m² the method is repeated first using a two-stage filtration of 0.1 - 10 mm pore size and if still no reduction is observed than the method is repeated using a single-stage dual-layer depth filter of 1 - 25 mm pore size for filtration. Using this approach, it is feasible to determine the disulfide bond reduction potential of the protein of interest via calculating the percentage of the intact form of the protein of interest or the percentage of the non-intact form of the protein of interest or calculating both. In addition, it has been shown that if this approach is used in a small scale setting the observed disulfide bond reduction corresponds to disulfide bond reduction on large scale.

Alternatively, if no reduction is observed with the charged non-woven filter with a membrane area in the range of between 0.004 and 0.500 m² the method is repeated with the charged non-woven filter with a membrane area in the range of between 0.004 and 0.500 m² at a higher pressure e.g. via blowdown of the filter after filtration *(cf.* Fig. 9). Using this approach, it is feasible to determine the disulfide bond reduction potential of the protein of interest via calculating the percentage of the intact form of the protein of interest or the percentage of the non-intact form of the protein of interest or calculating both. In addition, it has been shown that if this approach is used in a small scale setting the observed disulfide bond reduction corresponds to disulfide bond reduction on large scale.

Overall, it was demonstrated that a charged non-woven filter *e.g.* a charged non-woven filter with a membrane area in the range of between 0.004 and 0.500 m² induced a lower likelihood of disulfide bond reduction in a reference protein than a single-stage dual-layer depth filter of 1 - 25 mm pore size, or a two-stage dual-layer filtration combination of 0.1 - 10 mm pore size. Thus in case a given protein of interest does not show disulfide bond reduction using the method described herein employing a charged non-woven filter method is repeated with the same non-woven filter at higher pressure or using a one-stage dual layer depth filter of 1 - 25 mm pore size, or a two-stage filters of 0.1 - 10 mm pore size for filtration since the single-stage dual layer depth filter of 1 - 25 µm pore size, or a two-stage filter of 0.1 - 10 mm pore size for filtration were more likely to induce disulfide bond reduction in the assessed proteins i.e. monoclonal antibodies. On the other hand, if a given protein of interest already shows disulfide bond reduction as determined with the method described herein using a charged non-woven filter with a membrane area in the range of between 0.004 and 0.500 m² then it is deemed to be susceptible to disulfide bond reduction at large scale.

In one embodiment of the method described herein the method is used to assess whether for a given protein of interest at least one disulfide reduction prevention measure has to be implemented.

As appropriate mitigation measures for a reference protein are known, then it is possible to determine early on during process development which disulfide reduction prevention measures are needed to prevent disulfide bond reduction at full scale later on during clinical or commercial material production. This leads to a shorter process development time and lower costs associated with process development activities.

In one embodiment of the method described herein the at least one disulfide reduction prevention measure is selected from the group comprising:
- Using a synthetic charged non-woven fibrous chromatographic clarification media filter *e.g.* Harvest RC filter (3M)
- Chilling the bioreactor which has a temperature of 37 °C at the end of fermentation before starting the filtration procedure
- Maintaining a head space when storing cell culture fluid prior to filtration
- Addition of copper to the cell culture fluid prior to filtration
- Lowering of the pH of cell culture fluid prior to filtration
- Adding selenite to the cell culture fluid prior to filtration

In one example the method described herein was used for a monoclonal antibody - antibody C of Fig. 2 "AB C". AB C showed potential for disulfide bond reduction as determined with the method described herein *(cf.* Fig. 2). For AB C it was demonstrated that chilling as mitigation method to prevent disulfide bond reduction did not have a beneficial effect. Instead it was shown that for this particular clone expressing AB C maintaining a head space above the filtered cell culture fluid in the storage vessel prevented disulfide bond reduction *(cf.* Table 3). Hence not only the potential for disulfide bond reduction could be assessed and demonstrated on a small scale using the method described herein but also the mitigation measure was found on small scale to inhibit disulfide bond reduction, thereby saving time and costs associated with process development and manufacturing activities.

**Table 3 - Disulfide reduction mitigation measures for "AB C"**

| | % intact AB C | | |
|---|---|---|---|
| Time in h after O₂ displ. | Method carried out as described herein at ambient temperature | Method carried out as described herein, and kept at 2-8 °C | Method carried out as described herein at ambient temperature, following O₂ displacement samples were transferred to vessel with head space |
| 0 | 93,5 | 99,0 | 97,3 |
| 17 | 11,9 | 2,7 | 98,2 |
| 23 | 31,7 | 11,5 | 99,2 |
| 42 | 43,4 | 6,3 | 99,4 |
| 47 | 83,4 | 3,6 | 100,0 |
| 66 | 92,7 | 40,5 | 100,0 |
| 72 | 93,4 | 48,8 | 99,3 |

In one embodiment of the method described herein the method is used to compare at least two different clones of the mammalian cells expressing the protein of interest at a concentration within the range of between 0.2 g/l to 10 g/l for potential differences in the susceptibility of disulfide bond reduction in the expressed protein of interest, wherein the clones are from the same cell line and generated in the same way.

Clones can be selected that produce the protein of interest in a way that renders the protein of interest less susceptible to disulfide bond reduction, thereby minimizing the need for costly and tedious process development and even more costly and tedious reduction prevention measures during antibody production *(cf.* Table 4 were clone # 16 was chosen). Moreover, as process development time on identifying reduction-prone clone is shorter, therefore the lead time until a viable manufacturable antibody can be generated, cultured and filtered on production scale is thus optimized.

**Table 4 Clone Selection**

| % intact AB E | | |
|---|---|---|
| Time in h after O₂ displacement | Clone #16 | Clone #50 |
| 0 | 98,79 | 98,4 |
| 17 | 99,02 | 98,56 |
| 23 | 97,43 | 15,94 |
| 42 | 94,17 | 48,23 |

Method performed as described herein, cell culture fluid samples were filtered through a charged non-woven filter and O₂ was displaced as described in the example

A person skilled in the art knows how to generate a cell line expression a protein of interest.

In one embodiment of the method described herein the method is used to determine whether an accelerated processing is possible for a given protein of interest.

Such an accelerated processing could, for example, be achieved via starting filtration as well as subsequent purification steps e.g. protein An affinity chromatography as soon as enough cell culture fluid from a given cell culture has been harvested - *i.e.* is available - as opposed to waiting until all cell culture fluid from a given cell culture has been harvested before subsequent process steps are started. Apart from achieving an overall faster and thus more economic process accelerated processing also has the advantage that each portion of the cell culture fluid is stored for a shorter time period. Hence the time in which disulfide bond reduction can occur is also minimized which in turn increases step yields. Moreover, if such an accelerated process is combined with closed processing where the cell culture fluid is directly transferred from the cell culture vessel, to the filtration and then to the first purification step, process time can be even further shortened while also minimizing contamination risks.

Therefore, if it is known for a standard reference protein whether or not and under which conditions, respectively, an accelerated and possibly closed continuous harvesting, transfer and (Protein A) purification is possible, it is possible to determine for a new protein of interest - via comparison to the reference protein - whether an accelerated and possibly closed processing is possible thereby facilitating establishing an intensified (single-use) manufacturing system

### DESCRIBTION OF THE EMBODIMENTS

The various aspects of the invention described in this application are illustrated by the following example which is not meant to limit the invention in any way.

In this example the method described herein for assessing disulfide bond reduction potential of a protein of interest was carried out for two proteins of interest, here two different monoclonal antibodies expressed in CHO cells and are referred to therein as "AB A" and "AB B". The monoclonal antibody AB A was cultured on a 2 L scale and the culture resulted in 12.47 × 10⁶ cells per volumetric unit as determined by viable cell count using tryptophan blue staining. The monoclonal antibody AB B was cultured on a 2 L scale and the culture resulted in 12.84 × 10⁶ cells as determined by viable cell count using tryptophan blue staining.

The following filters were used Pall Filters (PDK5 SUPRAcap 50, CS050PDK5, 0.0022 m²) corresponding to single-stage dual-layer depth filter of 1 - 25 µm pore size and different 3M Filters - BC0025L05SP01A, 0.0025 m² BC0025L10SP02A, 0.0025 m² and BC0025L30SP02A, 0.0025 m² corresponding to two-stage depth filter of 0.1 - 10 µm pore size.

In a first step the filters were flushed with water for injection (WFI) in case of the Pall filters said flush was performed with no less than (NLT) 60 L/m² at 100 LMH for about 130 ml whereas for the 3M ZetaPlus filters (e.g. 05SP01A, 10SP02A and 30SP02A) the flush was performed with NLT 60 L/m² at 100 LMH for about 150 mL. The pressure during the flushing was kept below 1,4 bar (20 psi). Following the flushing the filters were pumped air dry. Then 200-250 ml of a Chinese Hamster Ovary cell culture fluid expressing the respective monoclonal antibodies "AB A" and "AB B" was filtered into 150 mL Sartorius Flexboy bags. Filtering was performed at 30, 50, and/or 70 LMH for the Pall filters and 100 LMH for 3M (ZetaPlus) filters. During filtering of cell culture fluid, the pressure was monitored in order not to exceed 1,4 bar (20 psi). Time, differential pressure and collection weight were recorded every 10 minutes for 30 LMH and 50 LMH sets, and every 5 minutes for 70 and 100 LMH sets. After the target collection volume of 220 ml was reached, the filters were pumped dry. Samples were collected and measured for turbidity, Dynamic Light Scattering (DLS) and O₂ level by BGA. While turbidity was assessed to acquire an indication on impurity components - the higher the turbidity the more impurity components are present in a sample - the DLS measurement was used to gauge the relative particle sizes of the present components. In addition, a sample of the non-filtered cell culture sample was aliquoted as untreated reference control. Subsequently the O₂ displacement was carried out. In order to do so, the filtered cell culture fluid samples were transferred to Thermo Scientific Nalgene Square PETG Media Bottles and flushed for 3 - 4 hours with N₂. The O₂ level was measured using a BGA analyzer and N₂ flushing was continued until the O₂ level was lower than 40 mmHg at 37 °C. Afterwards a 4.5 ml sample was taken of the O₂-displaced filtered cell culture sample at least once a day a day for no less than 72 hours after filtration. Each sample was split into 2 samples of 1,5 ml to which N-Ethylmaleimide (NEM) (final NEM concentration was 25 mM) was added and 2 samples of 0,5 ml each without NEM. The samples were stored at -70 °C for subsequent analysis. After all samples were taken the samples stored in NEM were processed further via purification on an protein A affinity resin in this case MabSelect SuRe LX resin or 50µL PreDictor MabSelect SuRe LX. In each case the sample eluate was no less than 2 mg/mL. Afterwards Caliper Analysis was performed. To do so the protein A eluate samples were diluted to 2 mg/mL in (Dulbecco's PBS) DPBS in a first 96-well plate thereby preparing the denaturing conditions samples. In a second 96-well plate protein A sample eluates were diluted in 2 mM NEM solution in 50 mM 2-(N-morpholino) ethanesulfonic acid (MES solution) to a final concentration of 1mg/ml ProA-purified mAb and incubated at room temperature for 30 min thereby preparing the native gel samples. Then the samples were added to a Caliper plate and the Caliper analysis was run according to the instructions of Perkin Elmer LabChip GXII User Manual. Finally, the samples were analyzed for intactness. The assessment was based on the fact that if the monoclonal antibody was intact, a 150 kDa tetrameric molecule (HHLL) was detected in the Caliper Analysis under non-denaturing/ non-reducing i.e. native conditions whereas if the monoclonal antibody assessed in this example was not intact (i.e. reduced) the Caliper analysis under non-reducing (native) conditions showed different proteins of different molecular weights, in this case 75 kDa for HL, 135 kDa for HHL. The denaturing gel on the other hand was used to demonstrate that the reason that the monoclonal antibody was not intact on the non-reduced (*i.e.* native) gel was due to disassembly of the heavy and light chains as supposed to incorrect or not-matching expression levels of heavy and light chains in the cell. The results in percentage of intactness are depicted in Fig. 2.

## Claims

1. Method for assessing disulfide bond reduction potential of a protein of interest comprising the following steps:
- Providing a cell culture fluid sample comprising mammalian cells expressing a protein of interest at a concentration within the range of between 0.2 g/l to 10 g/l
- Filtering said cell culture fluid sample over at least one filter
- Displacing O₂ in the filtered cell culture fluid sample
- Collecting at least one sample of the O₂-displaced filtered cell culture fluid sample
- Separating the proteins in said at least one O₂-displaced, filtered cell culture fluid sample according to their size under non-denaturing conditions
- Determining the disulfide bond reduction potential of the protein of interest.

2. Method according to claim 1 wherein the step of determining the disulfide bond reduction potential further comprises separating the proteins in said at least one O₂-displaced, filtered cell culture fluid sample according to their size under denaturing conditions.

3. Method according to claim 1 wherein prior to separating the proteins in said at least one O₂-displaced, filtered cell culture fluid sample according to their size under non-denaturing conditions and/or denaturing conditions the proteins in said at least one O₂-displaced filtered cell culture fluid sample are purified on affinity resin(s) or via ionic exchange chromatography.

4. Method according to claim 1 wherein another at least one other sample in addition to the at least one O₂-displaced filtered cell culture fluid sample is collected.

5. Method according to claim 1 wherein the step of determining the disulfide bond reduction potential of the protein of interest comprises calculating the percentage of the intact form of the protein of interest or the percentage of the non-intact form of the protein of interest or calculating both.

6. Method according to claim 4 wherein the step of determining the disulfide bond reduction potential further comprises comparing the percentage of intact form of the protein of interest or the percentage of the non-intact form of the protein of interest or both to a reference protein in order to assess the disulfide bond reduction potential of the protein of interest.

7. Method according to claim 1 wherein the filtration is carried out with a single-stage dual-layer depth filter of 1 - 25 µm pore size, or a two-stage depth filter of 0.1 - 10 µm pore size or a charged non-woven filter with a membrane area in the range of between 0.004 and 0.500 m².

8. Method according to claim 6 wherein if the one stage dual-layer depth filter is used the flux is set to at least 70 LMH (liters /m² per hour).

9. Method according to claim 7, wherein if no reduction is observed with the charged non-woven filter with a membrane area in the range of between 0.004 and 0.500 m² the method is repeated using a one stage dual-layer depth filter of 1 - 25 µm pore size, or a single-layer of 0.1- 10 µm pore size for filtration.

10. Use of the method according to claim 1 to assess whether for a given protein of interest at least one disulfide reduction prevention measure has to be implemented.

11. Use of the method according to claim 1 to compare at least two different clones of mammalian cells expressing the protein of interest at a concentration within the range of between 0.2 g/l to 10 g/l for potential differences in the susceptibility of disulfide bond reduction in the expressed protein of interest, wherein the clones are from the same cell line and generated in the same way.

12. Use of the method according to claim 1 to determine whether an accelerated processing is possible for a given protein of interest.
